# EUROPEAN PATENT APPLICATION

(11) **EP 2 312 551 A1**
(43) Date of publication of application: **20.04.2011**
(21) Application number: 09804731.9
(22) Date of filing: 04.08.2009
(51) Int. Cl.: G08G 1/16, A61B 3/113, A61B 5/0476, A61B 5/0484, A61B 5/18, B60R 1/00

(54) **DRIVER AWARENESS DEGREE JUDGMENT DEVICE, METHOD, AND PROGRAM**

(30) Priority: 05.08.2008 JP 2008201520
(71) Applicant: Panasonic Corporation, Kadoma-shi Osaka 571-8501 (JP)
(72) Inventor: MORIKAWA, Koji, Osaka 540-6207 (JP); NAKADA, Toru, Osaka 540-6207 (JP)
(74) Representative: Grünecker, Kinkeldey, Stockmair & Schwanhäusser Anwaltssozietät
(86) International application number: PCT/JP2009/003724
(87) International publication number: WO 2010/016244

(57) **Abstract**

Even when a driver is not directing his or her line of sight to objects in the surroundings, the amount of attention of the driver to the peripheral visual field can be determined, and safe driving assistance in accordance with the result of determination can be provided.

A driving attention amount determination apparatus includes: an electroencephalogram measurement section for measuring an electroencephalogram signal of a driver; an attention amount determination section for, from the electroencephalogram signal being measured based on a starting point which is a time point of occurrence of a visual stimulation occurring in the peripheral visual field of the driver, determining an amount of attention of the driver to the peripheral visual field; and an output section for calling attention of the driver by outputting a signal based on a result of the determination.

## Description

### TECHNICAL FIELD

The present invention relates to a technique of providing safe driving assistance by determining a state of a driver by utilizing an electroencephalogram.

### BACKGROUND ART

In recent years, in connection with accident prevention apparatuses related to automobile driving, methods of determining the state of a driver and providing driving assistance based on the result of determination are being studied. One visual perception function of a driver that is necessary for safe driving is detection of dangerous objects. Detection of dangerous objects involves noticing any dangerous motion of vehicles and pedestrians in the surroundings by peripheral vision. A deterioration in this detection function may lead to cross-collision accidents and rush-out accidents.

A "peripheral visual field" generally refers to a region spanning 130° in up and down directions and 180° in right and left directions, excluding a range of about 20° (central visual field) that is centered around a line of sight. As is known, in the peripheral visual field, it is difficult to recognize the shape and color of an object in detail, but sensitive response occurs with respect to any object that changes in time, e.g., a moving object or flickering light. In anticipation of a rushing out of a pedestrian or a motorcycle passing on the side, etc., a driver needs to pay attention to the peripheral visual field and any door mirrors or the like existing in this field. Therefore, when the amount of attention of the driver to the peripheral visual field becomes low, a remedy such as issuing an alarm to the driver is needed.

One method of determining the state of attention of a driver employs a camera which is aimed at the driver for detecting the line of sight and motions of the face of the driver, and determines an allocation of attention of the driver. For example, Patent Document 1 discloses a technique of determining the attention allocation of a driver by comparing a fixation point, which is detected from the line of sight and motions of the face of the driver, against an optimum fixation position that the driver should pay attention to, which is determined from the ambient situation of the driver's vehicle.

Another method determines the state of attention of a driver based on changes in the traveling velocity and the steering angle of the steering wheel and the like, which reflect the manner in which the driver's vehicle is being operated. For example, Patent Document 2 discloses a technique which determines a driver's degree of concentration on driving by using a brake response time with respect to a sudden deceleration of a preceding vehicle or the like, thus determining the level of need to output an alarm to the driver.

On the other hand, studies are under way to examine the amount of attention of a driver to driving by utilizing an event-related potential (ERP) of his or her electroencephalogram. An "event-related potential" refers to a transient potential fluctuation in the brain which occurs in temporal relationship with an external or internal event. Within an event-related potential, a positive component which appears near about 300 milliseconds based on the timing of an external visual stimulation or the like as a starting point is referred to as a P300 component, which is supposed to reflect perception of, or attention to, that stimulation.

For example, Non-Patent Document 1 discloses a study concerning the measurement of an amount of driving attention by utilizing an event-related potential. To specifically describe this study, in an experiment of trying to drive a vehicle so as to follow a preceding vehicle, the driver is asked to perform a task of stepping on a brake pedal of the driver's vehicle when brake lamps of the preceding vehicle are activated. Through a comparison of event-related potentials between the two experimental conditions of a travel (high-attention condition) during which the preceding vehicle applies sudden brakes and a travel during which this does not occur (low-attention condition), it reports that the amplitude of the P300 component of the event-related potential increases under the high-attention condition.

### CITATION LIST

### PATENT LITERATURE

Patent Document 1: Japanese Laid-Open Patent Publication No. 2004-178367
Patent Document 2: Japanese Laid-Open Patent Publication No. 2002-127780

### Non-PATENT LITERATURE

Non-Patent Document 1: "Technique for Measuring Driver's Attention Level by Using Event-Related Potentials", Ebe et al., Automotive Technologies, Vol.58, No.7, pp.91-96, 2004

### SUMMARY OF INVENTION

### TECHNICAL PROBLEM

However, the technique described in Patent Document 1 is based on the assumption that attention is not being paid to anywhere that the line of sight is not directed, and therefore cannot accurately determine the amount of attention of the peripheral visual field of the driver. For example, in an actual driving scene, while monitoring a preceding vehicle by central vision, the driver is simultaneously detecting the motions of flanking vehicles and pedestrians by peripheral vision, and determines the direction of his or her line of sight based on the situations of the front and the surroundings. Therefore, with the conventional technique, it is difficult to cope with the case where the line of sight is being directed to the front while also paying attention to the peripheral visual field, for example.

Moreover, in the technique described in Patent Document 2, since a brake response time with respect to a sudden deceleration of a preceding vehicle or the like is used, the derived degree of concentration on driving is limited to the front of the driver's vehicle, i.e., the central visual field of the driver. In an actual driving scene, it is very rarely the case that a response to an event occurring in the peripheral visual field of a driver is straightforwardly manifested in behavior such as braking. Therefore, with the conventional technique utilizing the manner in which the driver's vehicle is operated, the amount of attention of the driver to the peripheral visual field cannot be determined with a good accuracy.

Furthermore, in a study which is described in Non-Patent Document 1, an event-related potential (ERP) with respect to activation of the brake lamps of a preceding vehicle is similarly used. Therefore, the amount of driving attention being measured is limited to that pertaining to the central visual field of the driver, and it is impossible to measure the amount of attention to the peripheral visual field.

The present invention has been made in view of the aforementioned problems, and an objective thereof is to, even when a driver is not directing his or her line of sight to objects in the surroundings, determine the amount of attention of the driver to the peripheral visual field and provide safe driving assistance in accordance with the result of determination.

### SOLUTION TO PROBLEM

A driving attention amount determination apparatus according to the present invention comprises: an electroencephalogram measurement section for measuring an electroencephalogram signal of a driver; an attention amount determination section for determining an amount of attention of the driver to a peripheral visual field by utilizing an event-related potential in the electroencephalogram signal, the event-related potential being based on a starting point which is a time point of occurrence of a visual stimulation occurring in the peripheral visual field of the driver; and an output section for calling attention of the driver by outputting a signal based on a result of the determination.

The attention amount determination section may determine the amount of attention in accordance with an amplitude level of the event-related potential of the electroencephalogram signal based on a starting point which is the time point of occurrence of the visual stimulation.

The attention amount determination section may determine that the amount of attention is small if an amplitude of a P300 component of the event-related potential is smaller than a predetermined threshold value, the P300 component being a positive component in a zone from 300 milliseconds to 600 milliseconds based on a starting point which is the time point of occurrence of the visual stimulation.

The output section may output the signal to the driver when the attention amount determination section determines that the amount of attention is small.

The attention amount determination section may determine that the amount of attention is large if the amplitude of the P300 component of the event-related potential is greater than the predetermined threshold value; and when it is determined that the amount of attention is large, the output section may not output the signal to the driver.

The attention amount determination section may determine the amount of attention in accordance with a correlation coefficient between a prestored template and the electroencephalogram signal measured based on a starting point which is the time point of occurrence of the visual stimulation.

The output section may output at least one of: a video signal for presenting a text or a symbol on a screen for presenting information; and an audio signal to be output from a loudspeaker for outputting an audio.

The driving attention amount determination apparatus may further comprise a peripheral stimulation generation section for generating the visual stimulation in the peripheral visual field of the driver.

The driving attention amount determination apparatus may further comprise: an imaging section for capturing a video of a front of a vehicle being driven by the driver; and a peripheral stimulation detection section for, from the captured video, detecting the time point of occurrence of the visual stimulation occurring in the peripheral visual field, wherein, from the peripheral stimulation detection section, the attention amount determination section may receive information identifying the detected time point of occurrence of the visual stimulation.

The driving attention amount determination apparatus may further comprise a line-of-sight measurement section for measuring a line of sight of the driver, wherein the peripheral stimulation detection section may detect whether the visual stimulation has occurred in the peripheral visual field or not in accordance with the captured video and the line of sight of the driver at the time point of occurrence of the visual stimulation measured by the line-of-sight measurement section.

The driving attention amount determination apparatus may further comprise a situation detection section for detecting a velocity or head lamp activation of the vehicle, wherein, in accordance with a result of detection by the situation detection section, the peripheral stimulation detection section ma detect whether the visual stimulation is in the peripheral visual field or not.

If a difference in timing of occurrence between a visual stimulation detected in the peripheral visual field and a visual stimulation detected in the central visual field is equal to or less than a predetermined value, the attention amount determination section may exclude, from the subject of analysis, an event-related potential of the electroencephalogram signal with respect to the visual stimulation detected in the peripheral visual field.

The peripheral stimulation generation section may cause the visual stimulation in the peripheral visual field of the driver to be generated with a timing of occurrence having a difference from a timing of occurrence of the visual stimulation occurring in the central visual field of the driver, the difference being equal to or greater than a predetermined value.

A method of determining an amount of driving attention according to the present invention comprises the steps of: measuring an electroencephalogram signal of a driver; determining an amount of attention of the driver to a peripheral visual field by utilizing an event-related potential in the electroencephalogram signal, the event-related potential being based on a starting point which is a time point of occurrence of a visual stimulation occurring in the peripheral visual field of the driver; and calling attention of the driver by outputting a signal based on a result of the determination.

A computer program for determining an amount of driving attention according to the present invention, when executed by a computer, causes the computer to execute the steps of: receiving an electroencephalogram signal of a driver; determining an amount of attention of the driver to a peripheral visual field by utilizing an event-related potential in the electroencephalogram signal, the event-related potential being based on a starting point which is a time point of occurrence of a visual stimulation occurring in the peripheral visual field of the driver; and outputting a signal based on a result of the determination, thereby calling attention of the driver.

### ADVANTAGEOUS EFFECTS OF INVENTION

According to the present invention, from an electroencephalogram signal measured based on a starting point which is the time point of occurrence of a visual stimulation occurring in a peripheral visual field of a driver, an amount of attention of the driver to the peripheral visual field is determined. By using the electroencephalogram signal, it becomes possible to accurately determine an amount of attention to events that may possibly occur in the peripheral visual field of the driver (e.g., a sudden intrusion of a vehicle or a rushing out of a pedestrian), and based on the result of determination, a change in the state of the driver can be appropriately induced, e.g., by attention calling.

### BRIEF DESCRIPTION OF DRAWINGS

FIG. **1** is a diagram showing the functional block construction of a driving attention amount determination apparatus **100** according to the present invention, showing main constituent elements thereof.
FIG. **2** is a diagram showing the functional block construction of a driving attention amount determination apparatus **1** of Embodiment **1.**
FIG. **3** is a glasses-type head-mount display which combines a wearable-type electroencephalograph and a display is contemplated.
FIG. **4** is a diagram showing an example of a central visual field and a peripheral visual field in the case where an imaging section **15** is provided.
FIG. **5** is a diagram showing an example of a central visual field and a peripheral visual field in the case where a line-of-sight measurement section **18** is provided.
FIG. **6** is a flowchart showing a procedure of processing by a peripheral visual field attention amount determination section **13.**
FIG. **7** is a diagram showing an exemplary processing by the peripheral visual field attention amount determination section **13.**
FIG. **8** is a diagram showing an example of attention calling by an output section **14.**
FIG. **9** is a diagram showing a screen presented in an experiment performed by the inventors.
FIG. **10** is a diagram showing arithmetic mean waveforms for different visual fields and response times.
FIG. **11** is a diagram showing a relationship between visual fields and a maximum amplitude of a P300 component.
FIG. **12** shows diagrams showing probability distributions of the maximum amplitude of a P300 component in a non-cumulative electroencephalogram with respect to different visual fields.
FIG. **13** is a diagram showing the functional block construction of a driving attention amount determination apparatus **1** of Embodiment 2.
FIG. **14** is a flowchart showing a procedure of processing by a peripheral stimulation detection section **16.**
FIG. **15** is a diagram showing the functional block construction of the driving attention amount determination apparatus **1** in the case where a situation detection section **17** is provided in Embodiment 2.
FIG. **16** is a diagram showing an example of a central visual field and a peripheral visual field in the case where in the case where a situation detection section **17** is provided in Embodiment 2.
FIG. **17** is a diagram showing an example of a central visual field and a peripheral visual field in the case where a situation detection section **17** is provided in Embodiment 3.
FIG. **18** is a diagram showing the functional block construction of a driving attention amount determination apparatus **1** of Embodiment 3.
FIG. **19** is a diagram showing the functional block construction of the line-of-sight measurement section **18.**
FIG. **20(a)** is a diagram showing a data structure of calibration information of the line-of-sight measurement section **18** of Embodiment 3, and FIG. **20(b)** is a diagram showing an example of fixation position coordinates of a driver in a captured vehicle front video.
FIG. **21** is a diagram showing the functional block construction of a driving attention amount determination apparatus **1a,** which is obtained by introducing the line-of-sight measurement section **18** in the construction of Embodiment 1.
FIG. **22** is a diagram showing the functional block construction of a driving attention amount determination apparatus **2b,** which is obtained by introducing the situation detection section **18** in the construction of Embodiment 2.

### DESCRIPTION OF EMBODIMENTS

Hereinafter, with reference to the attached drawings, a concept of a driving attention amount determination apparatus according to the present invention will be described, followed by descriptions of Embodiments.

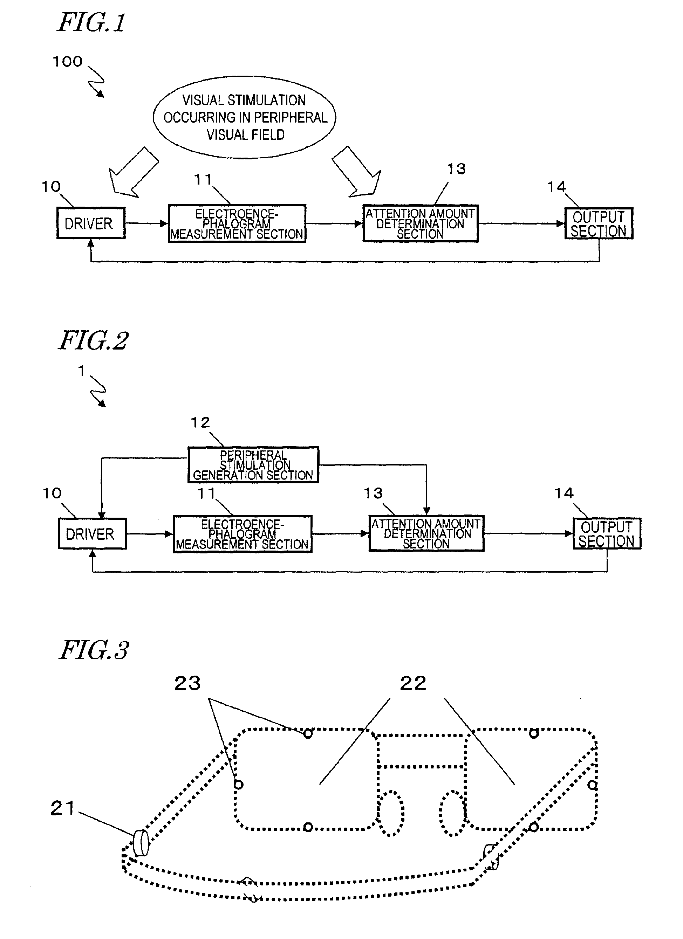

FIG. **1** is a block diagram showing main constituent elements of a driving attention amount determination apparatus **100** according to the present invention. The driving attention amount determination apparatus **100** includes an electroencephalogram measurement section **11,** an attention amount determination section **13,** and an output section **14.**

The electroencephalogram measurement section **11** measures an electroencephalogram signal from the driver **10.**

The attention amount determination section **13** determines an amount of attention of the driver **10** to the peripheral visual field, from an electroencephalogram signal which is measured based on a starting point which is the time point (or temporal point) of occurrence of a visual stimulation, the visual stimulation occurring in the peripheral visual field of the driver **10.**

As used herein, the "peripheral visual field " refers to a region of the visual field of a human excluding a certain visual field (central visual field) which is centered around the direction of a line of sight of the human. When contemplating a cone whose axis is the gaze direction of a human, the central visual field can be defined as a region which is contained within a certain angle constituted by the side face of this cone and the direction of the line of sight. In the following Embodiments, this certain angle is assumed to be about 20°.

The output section **14** outputs a signal based on the result of determination by the attention amount determination section **13,** thus calling attention of the driver **10.** As a result, the amount of attention of the driver is improved, thereby providing assistance in safe driving.

The aforementioned attention amount determination section **13** identifies the temporal point of occurrence of a visual stimulation occurring in the peripheral visual field. The visual stimulation may be provided by causing a light emission device which is provided in the driving attention amount determination apparatus **100** to emit light, or may be provided from the external environment (e.g., a lamp activated on another car). The following Embodiments will describe both cases.

Note that the specific construction of the electroencephalogram measurement section **11,** the attention amount determination section **13,** and the output section **14** will be described in more detail in the Embodiments.

### (Embodiment 1)

FIG. **2** shows a block construction diagram of a driving attention amount determination apparatus **1** according to the present embodiment.

The driving attention amount determination apparatus **1** is an apparatus which, by utilizing an electroencephalogram signal from the driver **10,** determines an amount of attention to driving and provides assistance in accordance with the result of determination. For example, an amount of attention to events that may possibly occur in the peripheral visual field of a driver (e.g., a sudden intrusion of a vehicle or a rushing out of a pedestrian) is determined by utilizing an electroencephalogram, and in accordance with the result of determination, attention of the driver is called.

The driving attention amount determination apparatus **1** includes an electroencephalogram measurement section **11,** a peripheral stimulation generation section **12,** an attention amount determination section **13,** and an output section **14.** The driver **10** block is illustrated for convenience of explanation.

The outline of the hardware construction and functions of each constituent element is as follows.

The electroencephalogram measurement section **11,** which is an electroencephalograph for example, measures an electroencephalogram of the driver **10.**

The peripheral stimulation generation section **12,** which is composed of LED light sources and a control circuit therefor, for example, generates a visual stimulation in the peripheral visual field of the driver **10.** The peripheral stimulation generation section **12** gives the visual stimulation to the driver **10,** and transmits information representing the timing of stimulation generation to the attention amount determination section **13.**

The attention amount determination section **13,** which is a microcomputer, for example, measures an electroencephalogram signal based on a starting point which is the temporal point of occurrence of the stimulation. The temporal point of occurrence of the stimulation is identified based on the information representing the timing of stimulation generation. From this electroencephalogram signal, the attention amount determination section **13** determines the amount of attention of the driver **10** to the peripheral visual field.

The output section **14** is a device which is capable of outputting at least one of an image and an audio. An image will be output by utilizing a display device such as a liquid crystal display device or a so-called organic EL display. An audio will be output by using a loudspeaker. The output section **14** calls attention of the driver **10** based on the result of determination.

Hereinafter, each constituent element will be specifically described.

By measuring potential changes at electrodes which are worn on the head of the driver **10,** the electroencephalogram measurement section **11** detects an electroencephalogram signal. The inventors envisage that a wearable-type electroencephalograph will be used in future. Therefore, the electroencephalograph may be a head-mount type electroencephalograph. It is assumed that the driver **10** has worn the electroencephalograph in advance.

Electrodes are disposed on the electroencephalogram measurement section **11** so that, when worn on the head of the driver **10,** the electrodes come in contact with the head at predetermined positions. The positions of the electrodes may be, for example, Pz (median parietal), A1 (earlobe), and the nasion as defined under the International 10-20 system. According to previous literature (Yo MIYATA et al., "New Physiopsychology", 1998, p. 119, Kitaoji Shobo), a P300 component of an event-related potential, which reflects perception of or attention to an external stimulation and appears near about 300 milliseconds based on the timing of occurrence of the stimulation as a starting point, is supposed to rise to its maximum amplitude at Pz (median parietal). However, P300 component measurement is also possible at Cz (epicranium) or Oz (occiput), which are in the neighborhood of Pz, and therefore electrodes may be disposed in these positions. These electrode positions are to be determined based on reliability of signal measurements, wearing ease, and the like.

As a result of this, the electroencephalogram measurement section **11** is able to measure an electroencephalogram of the driver **10.** The measured electroencephalogram is sampled so as to be computer-processible, and is sent to the peripheral visual field attention amount determination section **13.** Note that, in order to reduce the influence of noises mixing into the electroencephalogram, when the event-related potential is at issue, the electroencephalogram measured by the electroencephalogram measurement section **11** is previously subjected to a 15 Hz low-pass filtering process, for example.

The peripheral stimulation generation section **12** generates a visual stimulation in the peripheral visual field of a driver. Now, the definition of the peripheral visual field will be described by way of example.

For example, in the case of a glasses-type head-mount display (Head Mounted Display: HMD) which combines a wearable-type electroencephalograph and a display as shown in FIG. **3****,** a visual stimulation can be presented by flickering light sources **23,** e.g., LEDs, that are located at portions of the edges of a display **22.** In the present embodiment, it is assumed that the peripheral stimulation generation section **12** includes the light sources **23** and a control circuit (not shown) for controlling the flicker timing while supplying electric power to the light sources **23.**

As for the flickers serving as the visual stimulation, the number of flickers per unit time is determined from the determination accuracy, interval of determination, and the like of the attention amount determination section **13** described later. For example, in the case where a change in the amount of attention is to be determined every 3 minutes, if the number of electroencephalogram data that is required for determination (number of summations) is 30, then the number of flickers is 10 times per minute.

By combining various anti-noise measures and highly accurate analysis methods that are currently employed in the studies of the event-related potential (ERP) of electroencephalograms, the required number of flickers can be further reduced. The positions to be flickered may be randomly determined, or consecutive flickers may occur in a previously-determined order. The light sources **23** are to be placed in the peripheral visual field as viewed from the driver. Moreover, the light sources **23** (e.g., LEDs) may be positioned at edge portions of the windshield inside the vehicle, or on the door mirrors; in this case, too, the light sources **23** are to be placed in the peripheral visual field as viewed from the driver.

Moreover, the peripheral stimulation generation section **12** must generate a visual stimulation in the peripheral visual field at a different timing from any visual stimulation occurring in the central visual field of the driver. A specific example thereof will be described below.

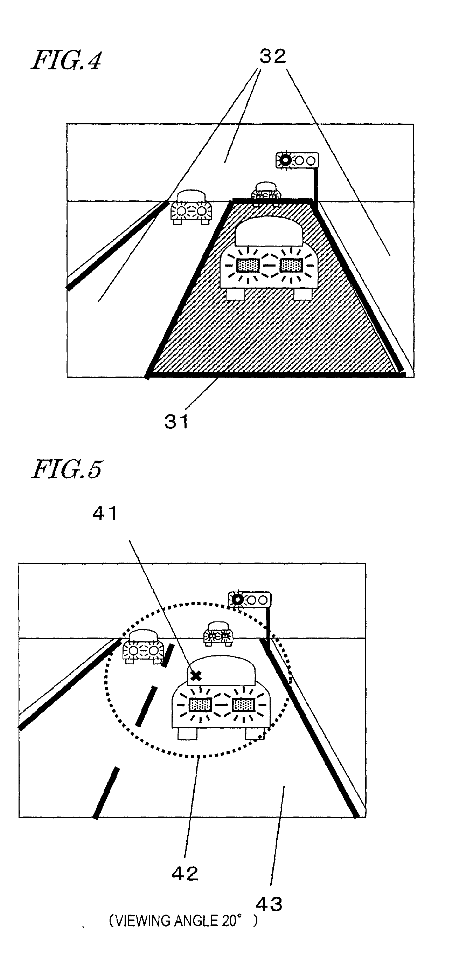

First, FIG. **4** shows an example of the central visual field **31.** Herein, within the field of vision of the driver, a region encompassing a lane in which the driver's vehicle exists and the front panel (not shown) is defined as the central visual field **31.** Accordingly, any region other than the central visual field **31** is defined as the peripheral visual field **32.**

In the case where the driver flickers a winker when changing the direction of travel or changing the lane, for example, a difference must be intentionally introduced between the timing of flickering a winker indication of the front panel existing in the central visual field **31** of the driver and the timing of flickering the light sources **23** existing in the peripheral visual field 32, which are disposed at edge portions of the windshield inside the vehicle, on the door mirrors, or the like.

The reason is as follows. The attention amount determination section 13 (described later) determines an amount of attention by using an event-related potential of the electroencephalogram based on a starting point which is the temporal point of occurrence of a stimulation, in particular, an event-related potential from 300 milliseconds to 600 milliseconds based on the temporal point of occurrence of a stimulation as a starting point. If visual stimulations simultaneously occur in both of the central visual field and the peripheral visual field, it is impossible to identify whether an amount of attention determined by the attention amount determination section 13 pertains to the central visual field 31 or to the peripheral visual field 32. Therefore, in order to ensure that the analysis time zones for the respective stimulations do not overlap each other, it is necessary for the peripheral visual field 32 to generate a visual stimulation with a predetermined time difference from any visual stimulation occurring in the central visual field **31.**

In the case of introducing a time difference between a visual stimulation in the peripheral visual field **32** and a visual stimulation occurring in the central visual field **31,** in order to be able to identify which one of the visual stimulations has induced the event-related potential (from 300 milliseconds to 600 milliseconds) that is currently at issue, it is necessary for the visual stimulation to be generated with a time difference of at least 300 milliseconds from the other visual stimulation. For example, when a winker on the front panel flickers every 600 milliseconds, the light sources **23** in the peripheral visual field may be flickered similarly every 600 milliseconds with a timing which is shifted by 300 milliseconds from the visual stimulation on the front panel.

Moreover, if visual stimulations are simultaneously detected in both of the central visual field **31** and the peripheral visual field **32,** or if the difference between the detection timings of the respective stimulations is 300 milliseconds or less, the amount of attention to the peripheral visual field **32** cannot be correctly measured as described above. In this case, the data of the event-related potential with respect to the stimulation generated in the peripheral visual field **32** is excluded from the subject of analysis by the attention amount determination section **13.** This process may be realized by the attention amount determination section **13** discarding the data without using it, or by the electroencephalogram measurement section **11** stopping the output of the electroencephalogram signal at that point in time.

Note that, generally speaking, a "peripheral visual field" refers to a region spanning 130° in up and down directions and 180° in right and left directions, excluding a range of about 20° (central visual field) that is centered around a line of sight (fixation point). Therefore, when providing a line-of-sight measurement section for measuring the line of sight of a driver, as shown in FIG. **5****,** a region which is within a viewing angle of 20° of the driver from a measured fixation point **41** may be defined as a central visual field **42,** and any other region (excluding a range of about 20°, centered around the line of sight) may be defined as a peripheral visual field **43.**

In the following description, it is assumed that the central visual field and the peripheral visual field are defined as shown in FIG. **4** and FIG. **5** described above. Then, assuming that the driving driver is basically looking in the front center, such that the central visual field and the peripheral visual field remain fixed.

However, in actual driving, the line of sight of the driver may fluctuate. Therefore, an instance of measuring the line of sight of the driver will be described later in another Embodiment. Moreover, an instance of utilizing an external visual stimulation will also be described later in another Embodiment.

The peripheral stimulation generation section **12** transmits information representing the point in time, or timing of occurrence (trigger), of the aforementioned stimulation to the attention amount determination section **13.**

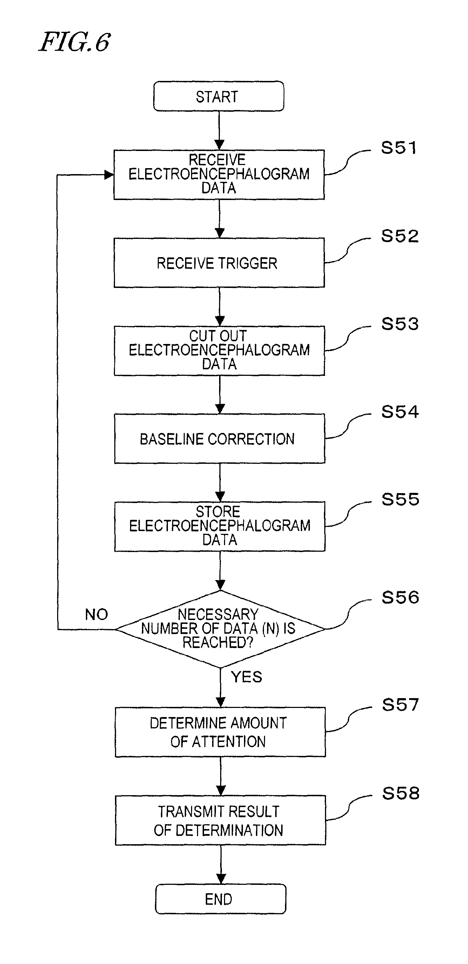

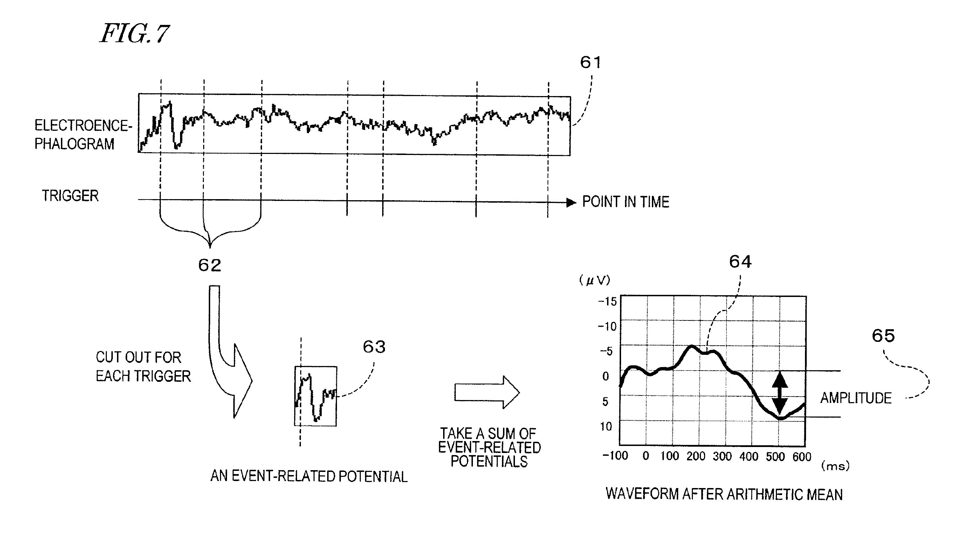

Based on the information received from the peripheral stimulation generation section **12,** the attention amount determination section **13** analyzes the measured electroencephalogram signal based on a starting point which is the temporal point of occurrence of a stimulation, thereby determining an amount of attention of the driver **10** to the peripheral visual field. Now, with reference to FIG. **6** and FIG. **7****,** the procedure of processing by the attention amount determination section **13** will be described.

FIG. **6** is a flowchart showing a procedure of processing by the attention amount determination section **13.** FIG. **7** shows exemplary waveforms concerning the processing by the attention amount determination section **13.**

At step **S51** in FIG. **6****,** the attention amount determination section **13** receives the measured electroencephalogram data from the electroencephalogram measurement section **11.** FIG. **7** shows the received electroencephalogram data **61.**

At step **S52,** from the peripheral stimulation generation section **12,** the attention amount determination section **13** receives information of the point in time at which a stimulation occurred. FIG. **7** shows such points in time **62** at which stimulations occurred, serving as triggers.

At step **S53,** in the electroencephalogram data received at step **S51,** the attention amount determination section **13** cuts out electroencephalogram data from 100 milliseconds to 600 milliseconds, based on each point in time of occurrence acquired at step **S52** as a starting point. FIG. **7** shows an example of electroencephalogram data (event-related potential) **63** that has been cut out. Note that the aforementioned time period in which to cut out the electroencephalogram data is predefined as a range which is certain to contain a P300 component of the event-related potential. The electroencephalogram data may be cut out in any time period other than this time period, so long as a P300 component is certain to be contained.

At step **S54,** the attention amount determination section **13** applies a baseline correction to the electroencephalogram data having been cut out. For example, a baseline correction is performed with respect to an average potential from -100 milliseconds to 0 milliseconds, based on the point in time at which the stimulation occurred as a starting point.

At step **S55,** the attention amount determination section **13** temporarily stores the electroencephalogram data which has been subjected to the baseline correction at step **S54.**

At step **S56,** the attention amount determination section **13** determines whether the number of electroencephalogram data stored at step **S55** has reached a predetermined, necessary number of summations to be made. If this value is not reached, the process returns to **S51;** if this value is reached, the process proceeds to **S57.**

Note that, in the studies of event-related potentials in general, an analysis is performed after deriving an arithmetic mean of electroencephalogram data. As a result, random action potentials of the brain that are not related to the event which is at issue are counteracted, thus making it possible to detect an event-related potential (e.g., a P300 component) that has a certain latency (i.e., the amount of time in which an action potential occurs since the starting point which is the temporal point of occurrence of a stimulation) and polarity.

For example, according to previous literature (Yo MIYATA et al., "New Physiopsychology, 1998, p. 110, Kitaoji Shobo), a process of taking 30 arithmetic means is performed.

In the present embodiment, the number of summations is 20 to 30, for example. By increasing this number, the SN ratio can be improved. However, this number of summations is only exemplary, and the present invention is not limited thereto. The amount of attention may be determined from a non-cumulative electroencephalogram (i.e., a single piece of electroencephalogram data).

At step **S57,** the attention amount determination section **13** performs an arithmetic mean process for the electroencephalogram data from the necessary number of times stored at step **S55.** FIG. **7** shows a waveform **64** and an amplitude **65** after taking the arithmetic mean.

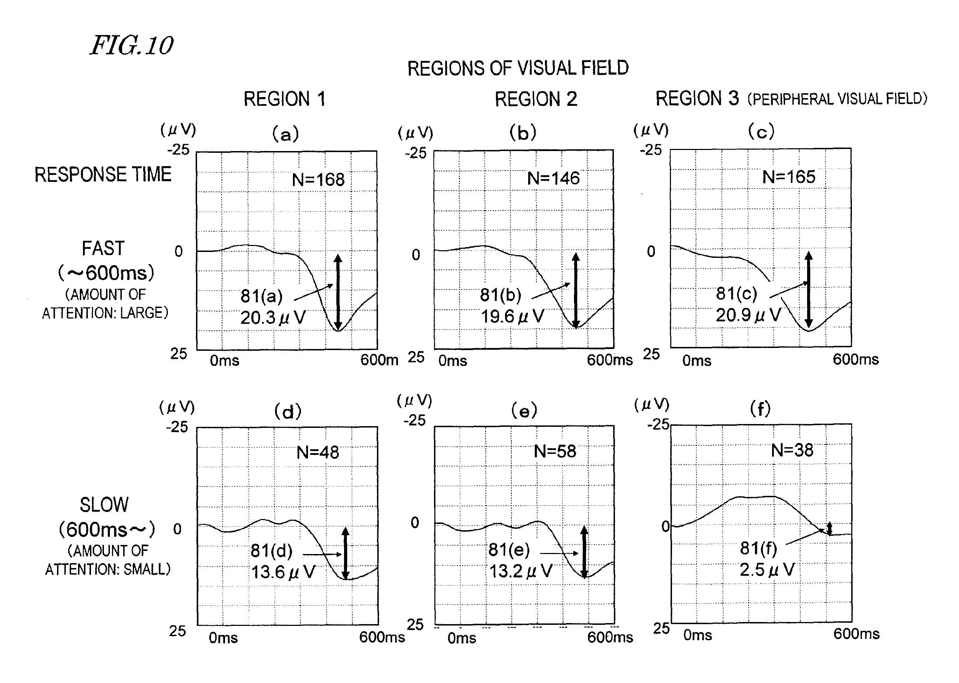

Furthermore, from the electroencephalogram data after taking the arithmetic mean, the amplitude of the event-related potential from 300 milliseconds to 600 milliseconds is analyzed, and based on whether the amplitude is large or small, a determination of an amount of attention is performed. At this time, based on ERP characteristics which are unique to the peripheral visual field identified by the inventors, the amount of attention to the peripheral visual field is determined. The details of the determination process will be described later with reference to the experimental results shown in FIGS. **10** to **12C.**

Now, the relationship between the range of electroencephalogram data whose sum is taken and the range of amount-of-attention determination is explained. For example, in the case where a sum of the electroencephalogram data for the flickering of all of the light sources **23** shown in FIG. **3** is taken, it is presumably an amount of attention to the entire peripheral visual field that is being determined. On the other hand, in the case where flickering is performed a necessary number of times for each light source position, and a sum of the electroencephalogram data for such flickering is taken with respect to each light source position, it is presumably an amount of attention with respect to each light source position that is being determined.

At step **S58** in FIG. **6****,** the attention amount determination section **13** transmits the aforementioned result of determination to the output section **14.**

The output section **14** presents a result of determination by the attention amount determination section **13** in the form of an image or an audio. Alternatively, based on the result of determination, the output section **14** may output a signal for calling attention of the driver from the apparatus side when the amount of attention is low. As a result, the amount of attention of the driver can be improved.

The signal(s) which is output from the output section **14** in order to call attention of the driver may be either one or both of a video signal and an audio signal, for example. Specifically, the driver may be spoken to with an audio signal, or an operating noise or an alarm sound may be presented with an audio signal; or with a video signal, a text or image presentation may be made on a car navigation system or a head-up display. As a result, it is possible to achieve an attention calling for improving the amount of attention of the driver.

The signals which the output section **14** outputs in order to call attention may include any control signal that causes an action for calling attention of the driver. Examples thereof include: a control signal for a direct information presentation using an AR (Augmented Reality) technique, such that an image is displayed in overlay on an object which needs attention; a control signal for causing an indirect intervention through a vibration of the steering wheel, a smell, or adjustment of an amount of fanned air; and the like. The actions for calling attention may include various actions such as the aforementioned examples. Any of the aforementioned examples can be considered as calling attention by exerting an external action to the driver.

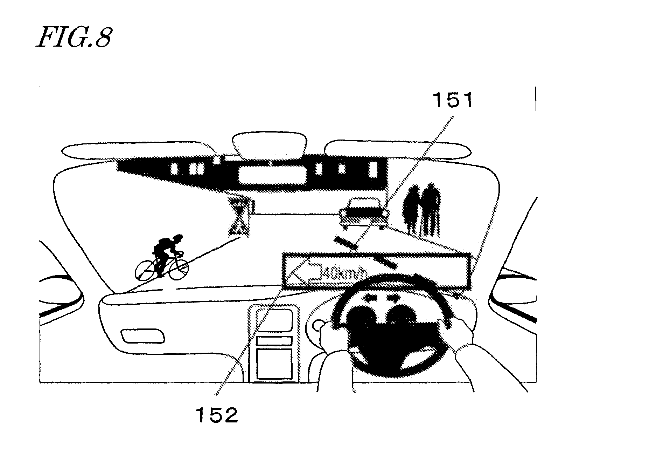

For example, FIG. **8** shows an example of attention calling by the output section **14.** This example is an exemplary indication in the case where, as a result of performing an electroencephalogram data summation and determining an amount of attention with respect to each light source position, the attention amount determination section **13** has determined that the amount of attention of the driver to the left side is deteriorated. In FIG. **8****,** an image signal for presenting a leftward arrow 152 on a head-up display (HUD) **151** is being output in order to call attention of the driver to the left side. This image signal functions as information for calling attention.

Now, results of an experiment performed by the inventors in connection with the aforementioned determination of an amount of attention will be described. Through an experiment described below, the inventors have found characteristics such that the amplitude of an event-related potential with respect to a stimulation occurring in the peripheral visual field from 300 milliseconds to 600 milliseconds greatly changes depending on whether the amount of attention is large or small.

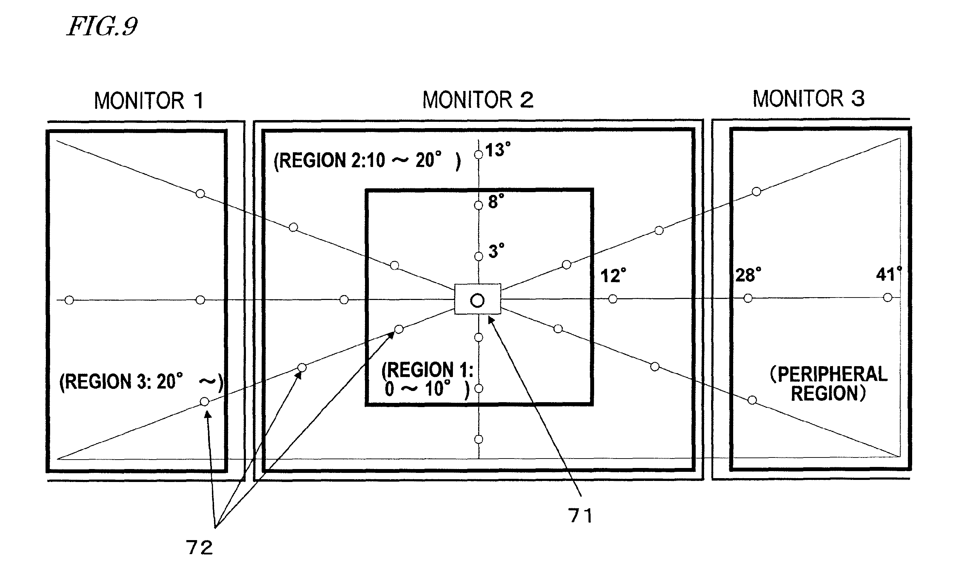

A total of 4 test subjects were involved, including one male and three females, with an average age of 21 ± 1.5 years. The experimental details will be described with reference to FIG. **9****.**

The inventors performed the experiment by a dual task method, in which each test subject was asked to perform two tasks concurrently. The first task was a central task 71 of counting to oneself a number of times that symbols (○/△/ □/×) presented on a screen center in FIG. 9 were switched. The second task was a peripheral task 72, in which lamps in the screen periphery were flickered in random order, and the test subject was supposed to press a button at hand as soon as noticing a flicker. The test subject was instructed to always keep his or her line of sight at the screen center. Thus, by having each test subject simultaneously perform the two tasks at the screen center and in the periphery, it is possible to examine how much attention is being paid to the periphery while also paying attention to the screen center. In order to allow the test subject to have a peripheral visual field, three 20" display monitors 1 to 3 were placed side by side, with a distance of 60 cm between the test subject and the screen. Although failing to mock a vehicle-driving environment, this experiment can be construed as an abstracted experiment for examining how quickly a change in the periphery can be noticed while watching the fixation point.

Each test subject was wearing an electroencephalograph (Polymate AP-1124 manufactured by TEAC Corporation), and the electrodes were positioned according to the International 10-20 electrode system, with a recording electrode at Pz (median parietal), a reference electrode at A1 (right earlobe), and a ground electrode at the metopic. Electroencephalogram data which was measured with a sampling frequency of 200 Hz and a time constant of 3 seconds were subjected to a bandpass filtering process from 1 to 6 Hz, and electroencephalogram data from -100 milliseconds to 600 milliseconds was cut out based on the flickering of a peripheral lamp as a starting point, and a baseline correction was performed with respect to an average potential from -100 milliseconds to 0 milliseconds.

FIG. **10** shows arithmetic mean waveforms of all test subjects, with respect to different combinations of first and second conditions, showing electroencephalogram data after the aforementioned processing was performed.

The first condition concerns a classification with respect to the visual fields. In this experiment, the classification was made as shown in FIG. **9****:** region 1 was defined as spanning a viewing angle (an angle at which a line connecting an eye position of a test subject and a fixation point at the screen center intersects a line connecting the eye position of the test subject and a flicker lamp) of equal to or greater than 0° but less than 10°; region 2 was defined as spanning a viewing angle of equal to or greater than 10° but less than 20°; and region 3 was defined as spanning a viewing angle of 20° or more.

The second condition concerns a classification with respect to each test subject's response time regarding a button press. In this experiment, in order to classify the amount of attention (large or small) as an experimental condition, a response time before achieving a button press was used. In physiopsychological experiments, response time is supposed to reflect the amount of attention; for example, in Patent Document 2, too, a degree of concentration of attention to driving is calculated by using a brake response time.

In this experiment, a relationship between an electroencephalogram and an amount of attention was analyzed, where the amount of attention served as an index of button-press response time. Among all response times in this experiment, very many samples were found between 400 milliseconds and 600 milliseconds. Therefore, a classification was made between: cases where a response was attained within 600 milliseconds (fast response time, i.e., a state of high attention to the stimulation); and cases where a response was not attained within 600 milliseconds (slow response time, i.e., a state of low attention to the stimulation). In each of the graphs in FIG. 10, the horizontal axis represents time (latency) since the lamp flickering at 0 milliseconds in units of milliseconds, and the vertical axis represents potential in units of µV. A number (N) shown in each graph represents each number of summations.

FIG. 10 indicates that, when the response time is fast, i.e., the amount of attention is large (**(a)** to **(c)** in FIG. 10), the amplitude of a P300 component (which is a positive component with a latency between 300 milliseconds and 600 milliseconds) is large regardless of the visual field. The maximum amplitudes (**81(a)** to **(c)**) of the P300 component in **(a)** to **(c)** of FIG. **10** are 20.3,µV 19.6µV, and 20.9µV, respectively. On the other hand, when the response time is slow, i.e., the amount of attention is small (**(d)** to **(f)** in FIG. **10**), the amplitude of the P300 component is relatively small. It can be seen that a particularly large decrease in the amplitude of the P300 component occurs in the case of region 3 with a viewing angle of 20° or more (a region which is generally considered as a peripheral visual field) combined with a small amount of attention (**(f)** in FIG. **10**). The maximum amplitudes (**81(d)** to **81(f)**) of the P300 component in **(d)** to **(f)** of FIG. **10** are 13.6µV, 13.2µV, and 2.5µV, respectively.

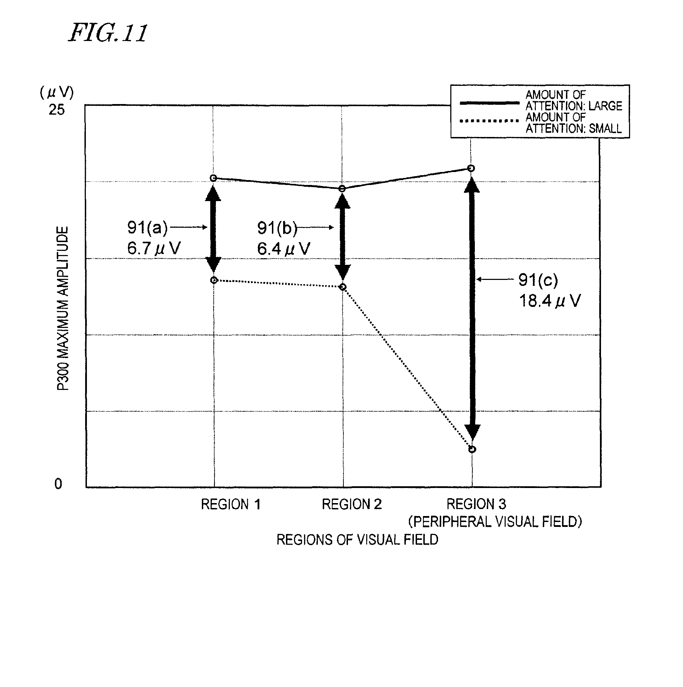

FIG. **11** shows the maximum amplitudes of a P300 component under the respective conditions of FIG. **10****.** The visual field (region 1/region 2/region 3) is taken on the horizontal axis, whereas the vertical axis represents potential in units of µV. The solid line represents the case where the amount of attention is large, whereas the dotted line represents the case where the amount of attention is small. In each visual field, the amplitude differences **91(a)** to **(c)** between the case of a large amount of attention and the case of a small amount of attention are 6.7µV, 6.4µV, and 18.4µV, respectively. FIG. **11** also indicates that, in region 3 with a viewing angle of 20° or more (peripheral visual field), thus indicative of considerable amplitude differences depending on whether the amount of attention is large or small.

Through determining the amplitude level of an event-related potential by utilizing the aforementioned ERP characteristics in the peripheral visual field, it becomes possible to accurately determine an amount of attention to an event that may occur in the peripheral visual field of a driver, e.g., a sudden intrusion of a vehicle or a rushing out of a pedestrian, based on an electroencephalogram.

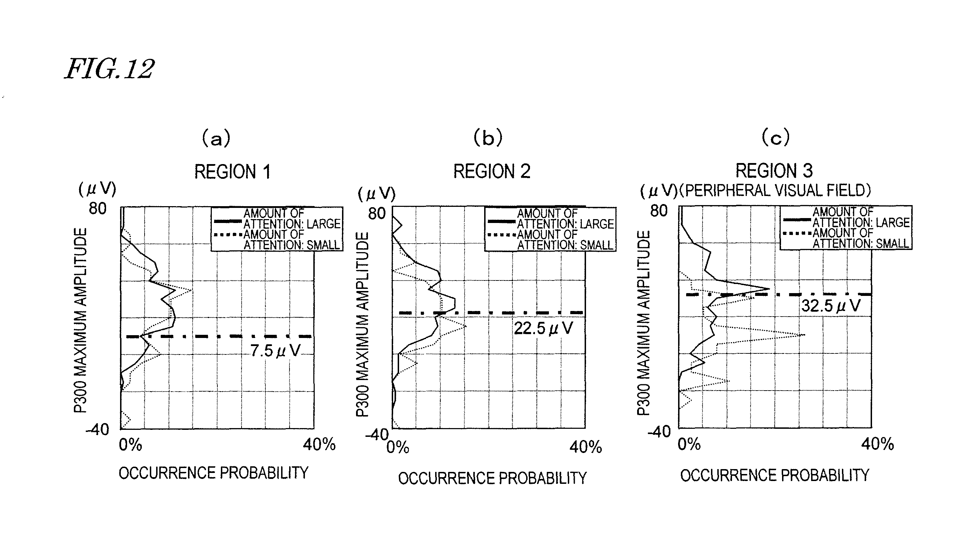

Furthermore, advantages of the construction according to the present embodiment will be specifically described based on results of a trial calculation of an attention amount distinction rate in this experiment. FIGS. **12(a)** to **12(c)** show probability distributions of the maximum amplitude of a P300 component in a non-cumulative electroencephalogram with respect to different visual fields. FIG. **12(a)** shows a probability distribution for region 1; FIG. **12(b)** shows that for region 2; and FIG. **12(c)** shows that for region 3 (peripheral visual field). In each graph, the vertical axis represents potential in units of µV, and the horizontal axis represents occurrence probability for the respective amounts of attention in units of %. Moreover, Table 1 shows a distinction rate when making a determination as to whether the amount of attention is large or small in each visual field.

**[Table 1]**

| | (a) region 1 | (b) region 2 | (c) region 3 |
|---|---|---|---|
| attention amount distinction rate | 55.4% | 59.8% | 73.1% |

In the method of distinction, a threshold value of ERP maximum amplitude that maximizes the distinction rate in each visual field is chosen, and a determination as to whether the amount of attention is large or small is made based on whether or not the ERP amplitude of each non-cumulative electroencephalogram is equal to or greater than this threshold value. As the threshold value that maximizes the distinction rates, a threshold value is chosen at which a largest average between the correctness rate of the case where the amount of attention is large and the correctness rate of the case where the amount of attention is small is obtained. In the cases of FIGS. **12(a)** to **12(c)****,** the aforementioned threshold values were 7.5µV, 22.5µV, and 32.5µV, respectively. The threshold values are indicated by dot-dash lines in FIGS. **12(a)** to **12(c)****.**

From FIGS. **12(a)** to **12(c)** and Table 1, it can be seen that, in the case of region 1 of FIG. **12(a)** and region 2 of FIG. **12(b)****,** there is a considerable overlap between the probability distribution of the case where the amount of attention is large and the probability distribution of the case where the amount of attention is small, and that the attention amount distinction rates are as low as 55.4% and 59.8%.

On the other hand, in region 3 (peripheral visual field) with a viewing angle of 20° or more of FIG. **12C****,** there is a certain degree of separation between the probability distribution of the case where the amount of attention is large and the probability distribution of the case where the amount of attention is small, and the attention amount distinction rate is 73.1%, which is a quite high value for a determination using a non-cumulative electroencephalogram. Thus, with the amount-of-attention determination in the peripheral visual field according to the present embodiment, a high distinction rate can be constantly obtained with a non-cumulative electroencephalogram, without having to perform a summation on the order of tens to hundreds of times. In other words, rather than determining a state of attention over a time period spanning about several minutes, the amount of attention of the driver at a given moment can be determined.

Instead of employing threshold processing with respect to the amplitude of an event-related potential as described above, the amount of attention to the peripheral visual field may be determined based on correlation coefficient values with respect to prestored templates. Herein, the templates are meant to be the arithmetic mean waveform data of the electroencephalogram signal **(c)** in the case where the amount of attention is large and the arithmetic mean waveform data of the electroencephalogram signal **(f)** in the case where the amount of attention is small, both pertaining to region 3 (peripheral visual field) in FIG. **10****.** Correlation coefficients (e.g., Pearson product-moment correlation coefficients) between each non-cumulative electroencephalogram data and the two templates may be calculated, and if the correlation coefficient with the electroencephalogram data of **(c)** has a larger value, then the amount of attention may be determined as large, and if the correlation coefficient with the electroencephalogram data of **(f)** has a larger value, then the amount of attention may be determined as small. By using this determination method based on templates, it becomes possible to achieve a finer analysis and determination which is based not only on the maximum amplitude values of event-related potentials, but also on information concerning waveform shapes.

With the construction and procedure of processing according to the present embodiment, in an apparatus which provides safe driving assistance by determining the state of a driver, a visual stimulation is generated in the peripheral visual field of the driver, and the amount of attention of the driver to the peripheral visual field is determined from an event-related potential of an electroencephalogram signal based on the temporal point of occurrence of the stimulation as a starting point. This makes it possible to determine an amount of attention with respect to an event that may possibly occur in the peripheral visual field of the driver, e.g., a sudden intrusion of a vehicle or a rushing out of a pedestrian, even in the absence of any behavior index such as braking. Then, based on the result of determination, assistance for inducing a change in the state of the driver, e.g., attention calling, can be appropriately provided.

### (Embodiment 2)

A driving attention amount determination apparatus according to the present embodiment includes an imaging section for imaging the front of a driver's vehicle. From the video that is captured by the imaging section, the driving attention amount determination apparatus detects an occurrence of a visual stimulation, which serves as a starting point when analyzing an event-related potential of the electroencephalogram, and discerns a central visual field and a peripheral visual field from the position in the captured video at which the visual stimulation occurred. Then, an amount of attention to the peripheral visual field is determined.

As a result, without having to purposely provide a visual stimulation from the driving attention amount determination apparatus as in Embodiment 1, it is possible to determine an amount of attention to the peripheral visual field by taking from within the captured video of the front a natural visual stimulation that occurs in front of the driver during driving.

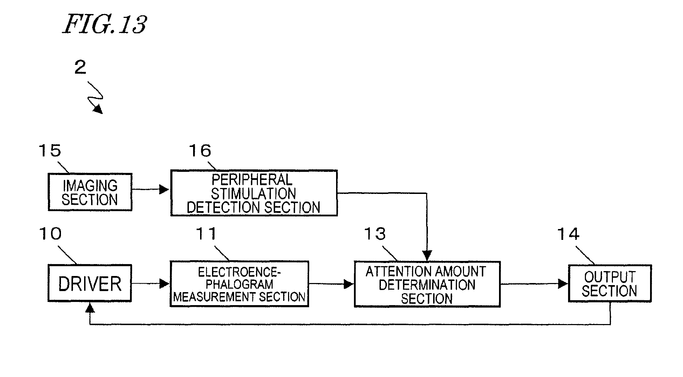

FIG. **13** shows a block construction diagram of the driving attention amount determination apparatus **2** according to the present embodiment. The driving attention amount determination apparatus **2** differs from the driving attention amount determination apparatus **1** of Embodiment 1 (FIG. **2**) in that, the driving attention amount determination apparatus **2** further includes the imaging section **15** in addition to the construction of the driving attention amount determination apparatus **1,** and that the peripheral stimulation generation section **12** of the driving attention amount determination apparatus **1** is replaced by a peripheral stimulation detection section **16.** Hereinafter, the differing constituent elements will be specifically described.

The imaging section **15** is a camera which is capable of moving picture imaging, for example. The imaging section **15** is disposed at the vehicle front (on the dashboard, behind the rear-view mirror, etc.), and images the vehicle front with an angle of view of 105° along the vertical direction and 135° along the lateral direction, at 30 frames per second, for example. The imaging section **15** can capture an image such as that shown in FIG. **4****.**

From the video captured by the imaging section **15,** the peripheral stimulation detection section **16** detects the temporal point of occurrence of a visual stimulation to serve as a starting point when analyzing an event-related potential of the electroencephalogram, and simultaneously determines (identifies) a region of the captured video at which the visual stimulation has occurred. As used herein, a visual stimulation is anything of which an amount of change of the luminance in the video exceeds a predetermined threshold value. Note that it is only exemplary to employ an amount of change. For example, a rate of change in luminance may be employed; in this case, an occurrence of a visual stimulation may be determined when there is a rate of change in luminance of 50% or more. For example, brake lamps of a preceding vehicle, a winker of a flanking vehicle, head lights of an oncoming vehicle, switching of a traffic light, and the like may be visual stimulations. The point in time at which any such change has occurred is detected as the temporal point of occurrence of a visual stimulation.

The peripheral stimulation detection section **16** detects the temporal point of occurrence of a visual stimulation as defined above, and determines whether the position of the stimulation, i.e., the position of luminance change, is in the central visual field or the peripheral visual field. As the determination method, any stimulation that exists in the region of the lane in which the driver's vehicle is present in the captured video as shown in FIG. **4** is determined as being in the central visual field **31,** whereas any stimulation that exists in anywhere other than the aforementioned region is determined as being in the peripheral visual field **32.** If the stimulation is determined as being in the peripheral visual field **32,** the temporal point of occurrence of the stimulation is transmitted to the attention amount determination section **13.**

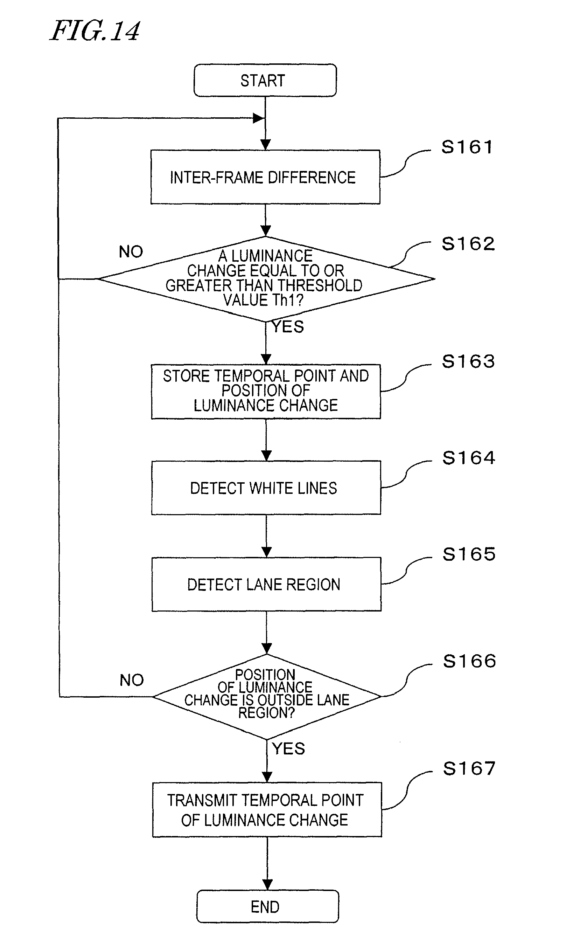

Hereinafter, with reference to FIG. **14****,** a procedure of processing by the peripheral stimulation detection section **16** will be described. FIG. **14** is a flowchart showing a procedure of processing by the peripheral stimulation detection section **16** according to the present embodiment. In the following description, an amount of change in luminance will be taken for example.

At step **S161,** in the vehicle front video having been captured by the imaging section **15,** the peripheral stimulation detection section **16** calculates a luminance image difference between frames.

At step **S162,** from the aforementioned difference, the peripheral stimulation detection section **16** determines whether or not a luminance change has occurred that is equal to or greater than a predetermined threshold value Th1. If such a luminance change has occurred, control proceeds to step **S163;** if not, control returns to step **S161** to calculate a next inter-frame luminance difference.

At step **S163,** the peripheral stimulation detection section **16** stores the temporal point of luminance change and the position at which the luminance change occurred in that image.

At step **S164,** the peripheral stimulation detection section **16** detects white lines from the inter-frame luminance difference calculated at step **S161.** Specifically, in a road image taken from a vehicle which is traveling at a certain rate, the asphalt on the road surface and the structures and vegetation around the road appear to be moving, but the white lines, which maintain essentially constant luminance values in their places, appear still in the image. Therefore, from the inter-frame luminance difference, the peripheral stimulation detection section **16** detects any region which is equal to or less than a predetermined threshold value Th2 to be a region of a non-moving white line.

At step **S165,** by using the detected white lines, the peripheral stimulation detection section **16** extracts, as a lane region, a region where the distance between both white lines is equal to or greater than a certain width. In the example of FIG. **4****,** the lane region is shown as the central visual field **31.**

At step **S166,** the peripheral stimulation detection section **16** determines whether the position of luminance change stored at step **S163** falls outside the lane region extracted at step **S165** or not. If it is determined as falling outside the lane region, the luminance change is determined to have occurred in the peripheral visual field **32** (FIG. **4****),** and control proceeds to step **S167.** If it is determined as not being outside the lane region, the luminance change is determined to have occurred in the central visual field **31** (FIG. **4****),** and control returns to step **S161** to calculate a next inter-frame luminance difference.

At step **S167,** to the attention amount determination section **13,** the peripheral stimulation detection section **16** transmits the temporal point of luminance change which has been determined as a luminance change in the peripheral visual field **32** (FIG. **4****).**

The above description assumes that the peripheral visual field **31** and the peripheral visual field **32** do not significantly change, but are substantially fixed.

However, the central visual field and the peripheral visual field of a driver are actually not fixed, and will presumably change depending on the driving situation (velocity of the driver's vehicle or lightness of the neighborhood of the driver's vehicle). For example, when the driver's vehicle is traveling on an expressway at 100 km per hour or more, the field of vision of the driver is narrower than when the vehicle is stopped. Moreover, when the neighborhood of the driver's vehicle is dark, e.g., at night, the field of vision of the driver is narrower than in the daytime. When the field of vision of the driver becomes narrow, detection of dangerous objects is delayed even in a visual field which is closer to the center, thus increasing the possibility of cross-collision accidents and rush-out accidents.

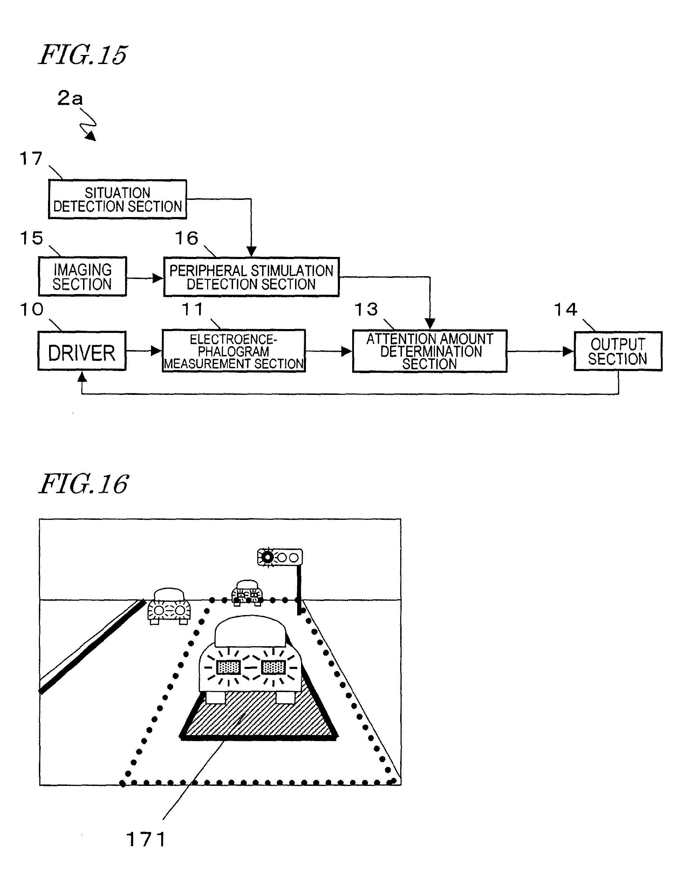

Therefore, by detecting the driving situation and changing the central visual field and peripheral visual field, an amount-of-attention determination which is truer to the actual circumstances is made possible. With reference to FIG. **15****,** a variant of the driving attention amount determination apparatus **2** according to the present embodiment will be described.

FIG. **15** shows the construction of a driving attention amount determination apparatus **2a** which includes a situation detection section **17.** For example, the situation detection section **17** is connected to a speedometer of the vehicle, a sensor which is provided for an autolight function of automatically activating the head lamps when it becomes dark, and/or an activation switch of the head lamps, thus detecting the driving situation of the driver's vehicle (e.g., velocity, lightness of the neighborhood of the driver's vehicle and/or activation-inactivation of the head lamps of the driver's vehicle). Based on the detected situation, the central visual field is defined so as to be more constricted when traveling at high speed or at night, etc. than when stopped or in the daytime. By defining any region other than the central visual field as a peripheral visual field, an amount of attention can be determined.

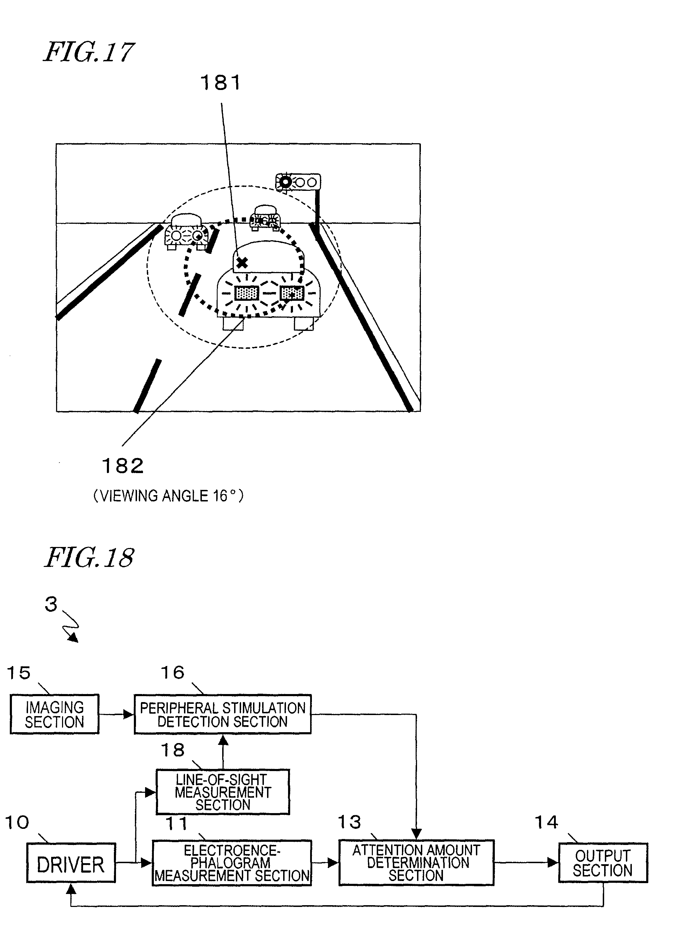

FIG. **16** and FIG. **17** respectively show constricted central visual fields **171** and **182.** This makes it possible to set a visual field which accommodates changes in the field of vision of the driver occurring due to changes in the external situation. As a result, it is possible to detect an amount of attention to the peripheral visual field in accordance with the velocity of the driver's vehicle and the activation/inactivation of the head lamps of the driver's vehicle, thus reducing the danger of cross-collision accidents and rush-out accidents.

In accordance with the velocity of the driver's vehicle and the activation/inactivation of the head lamps of the driver's vehicle as detected by the situation detection section **17,** the peripheral stimulation detection section **16** changes the definitions of the central and peripheral visual fields. FIG. **16** shows the constricted central visual field **171.**

Table 2 shows an exemplary relationship between the velocity of the driver's vehicle and an area ratio of the central visual field relative to that when the vehicle is stopped.

**[Table 2]**

| | (a) | (b) | (c) |
|---|---|---|---|
| | ∼50 km/hour | 50∼100 km/hour | 100 km/hour ∼ |
| Area ratio of central visual field | 1 | 0.8 | 0.6 |

In the above table, (a) at less than 50 km per hour, the area ratio relative to that when the vehicle is stopped is set to 1; (b) at 50 km per hour or more but less than 100 km per hour, the area ratio is set to 0.8; and (c) at 100 km per hour or more, the area ratio is set to 0.6. In FIG. **16****,** the area ratio of the central visual field **171** relative to the central visual field when the vehicle is stopped is 0.8. This makes it possible to determine an amount of attention to the peripheral visual field in accordance with the velocity of the driver's vehicle, thus reducing the danger of cross-collision accidents and rush-out accidents.

Table 3 shows an exemplary relationship between activation/inactivation of the head lamps of the driver's vehicle and an area ratio of the central visual field relative to that in the daytime.

**[Table 3]**

| | (a) | (b) | (c) |
|---|---|---|---|
| | no lamps are activated | small lamps are activated | head lamps are activated |
| area ratio of central visual field | 1 | 0.8 | 0.6 |

In the above table, (a) when no lamps are activated, the area ratio relative to that in the daytime is set to 1; (b) when the small lamps (clearance lights) are activated, the area ratio is set to 0.8; and (c) when the head lamps (head lights) are activated, the area ratio is set to 0.6. This makes it possible to determine an amount of attention to the peripheral visual field in accordance with activation/inactivation of the head lamps of the driver's vehicle, thus reducing the danger of cross-collision accidents and rush-out accidents.

With the construction and procedure of processing according to the present embodiment, occurrence of a visual stimulation is detected from within a captured video of the front of the driver's vehicle; the central visual field and the peripheral visual field are discerned from the position in the captured video at which the stimulation occurred; and an amount of attention to the peripheral visual field is determined. Thus, without having to purposely provide a visual simulation from the driving attention amount determination apparatus, it is possible to determine an amount of attention to the peripheral visual field by taking from within the captured video of the front a natural visual stimulation that occurs in front of the driver during driving. Furthermore, it is possible to determine an amount of attention to the peripheral visual field in accordance with the driver's vehicle and the surrounding situation.

### (Embodiment 3)

In Embodiment 2, a region in which a stimulation occurs is determined based on the assumption that a driver is basically looking in the front center during driving. However, the driver may not always have his or her line of sight directed in the front center when a visual stimulation occurs, and therefore the peripheral visual field may always be fluctuating.

Therefore, in the present embodiment, a line-of-sight measurement section for measuring the line of sight of the driver is provided in the driving attention amount determination apparatus. The driving attention amount determination apparatus determines a region in which a visual stimulation occurs in accordance with the position of a fixation point of the driver.

FIG. **18** shows a block construction diagram of a driving attention amount determination apparatus **3** according to the present embodiment. The driving attention amount determination apparatus **3** includes a line-of-sight measurement section **18** in addition to the construction of the driving attention amount determination apparatus **2** (FIG. **13****).**

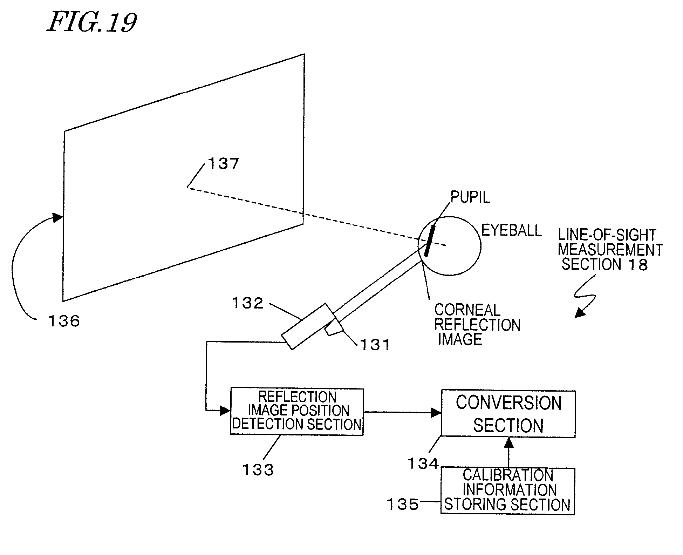

FIG. **19** shows an exemplary construction of the line-of-sight measurement section **18.** The line-of-sight measurement section **18** measures a fixation point **137** of the driver on a two-dimensional plane **136** which is a projection of a view in the vehicle front (i.e., a vehicle front video being captured by the imaging section **15).** Specifically, in the line-of-sight measurement section **18,** a near-infrared light source **131,** which is a point light source, irradiates the eyeballs with near-infrared light, and a video of the eyeballs is captured with a CCD camera **132.** Then, by using the captured video, a reflection image position detection section **133** detects the position of a corneal reflection image of the light source at the pupil and/or the cornea surface. A calibration information storing section **135** stores in advance a relationship between corneal reflection image positions and fixation point coordinates in the vehicle front video captured by the imaging section **15.** Based on the calibration information, a conversion section **134** measures a fixation point of the driver in the vehicle front video from the position of the corneal reflection image.

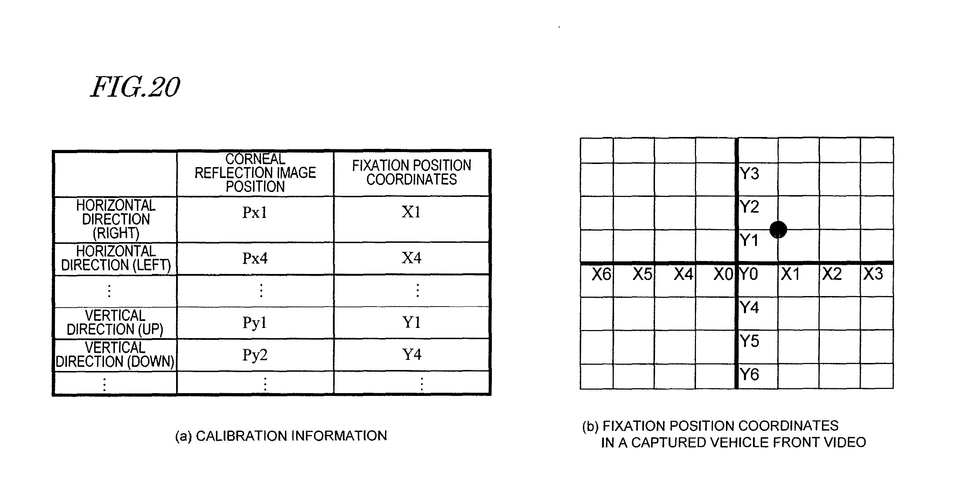

FIG. **20(a)** shows an example of calibration information, and FIG. **20(b)** shows exemplary coordinates of a fixation position in the vehicle front video. The calibration information is composed of corneal reflection image positions and fixation position coordinates. The conversion section **134** converts the corneal reflection image position (Pxn,Pyn) detected by the reflection image position detection section **133** into fixation position coordinates (Xn,Yn) of the driver in the vehicle front video.

The line-of-sight measurement section **18** may be a head-mount type measuring instrument which is worn by the driver in advance, or an onboard-type measuring instrument which is disposed near the rear-view mirror of the vehicle.

The peripheral stimulation detection section **16** detects the temporal point of occurrence of a visual stimulation, and determines whether the position of the stimulation is in the central visual field or the peripheral visual field. As the determination method, the region in which the stimulation has occurred is determined based on the position of the fixation point **41** (FIG. **5****)** as measured by the line-of-sight measurement section **18.** As described above, a peripheral visual field generally refers to a region spanning 130° in up and down directions and 180° in right and left directions, excluding a range of about 20° (central visual field) that is centered around a line of sight. Therefore, as shown in FIG. **5****,** any stimulation existing in a region which is within a viewing angle of 20° of the driver from the measured fixation point **41** is determined as being in the central visual field **42,** and any stimulation existing in anywhere other than the aforementioned region is determined as being in the peripheral visual field **43.** Then, if the stimulation is determined as being in the peripheral visual field **43,** the temporal point of occurrence thereof is transmitted to the attention amount determination section **13.**

With the construction and procedure of processing according to the present embodiment, the line of sight of a driver is measured, and a peripheral visual field is determined in accordance with the position of a fixation point, thus making it possible to accurately determine whether the stimulation is in the peripheral visual field or not even if the driver is not directing his or her line of sight in the front center when the visual stimulation occurs. As a result, an amount of attention to the peripheral visual field can be determined with a higher accuracy.

In the case where the driving attention amount determination apparatus according to the present invention is implemented as a head-mount display type apparatus to be worn by a user, an amount of attention to the peripheral visual field can be determined even while the user is riding a bicycle or walking, without being limited to safety assistance while driving an automobile. For example, when the user is watching television on a sub-screen of a wearable-type display while walking, an amount of attention to the peripheral visual field of the user may be determined based on an event-related potential of his or her electroencephalogram, thus making it possible to appropriately call attention to an obstacle in walking, etc.

The aforementioned line-of-sight measurement section **18** may be provided in the driving attention amount determination apparatus **1** (FIG. **2****)** of Embodiment 1 and the driving attention amount determination apparatus **2a** (FIG. **15****)** of Embodiment 2.

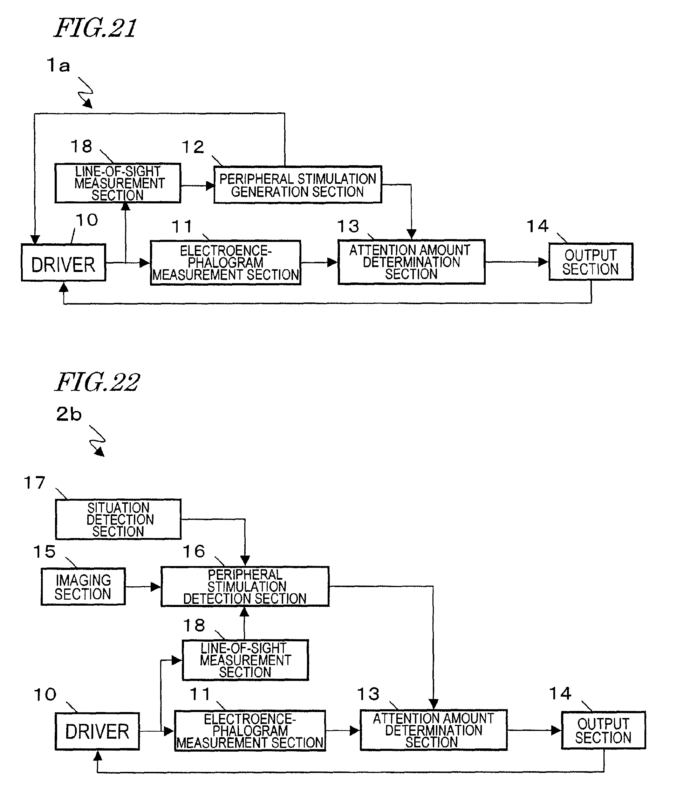

For example, FIG. **21** shows a block construction diagram of a driving attention amount determination apparatus **1a** according to a variant of Embodiment 1. The driving attention amount determination apparatus **1a** further includes the line-of-sight measurement section **18** in addition to the construction of the driving attention amount determination apparatus **1.** As the line-of-sight measurement section **18,** the construction shown in FIG. **19** described above can be adopted. Hereinafter, differences in functions and operation of the driving attention amount determination apparatus **1a** from the driving attention amount determination apparatus **1** (FIG. **2****)** will be described.

By providing the line-of-sight measurement section **18** in the driving attention amount determination apparatus **1a,** it becomes possible for the driving attention amount determination apparatus **1a** to dynamically identify the incessantly-changing central visual field and peripheral visual field of a driver. Thus, the peripheral stimulation generation section **12** is able to selectively flicker a light source which is positioned in the peripheral visual field of the driver.

Taking the glasses-type head-mount display of FIG. 3 for example, if the line-of-sight measurement section **18** finds that the line of sight of the driver is directed toward the left side, the peripheral stimulation generation section **12** can present a visual stimulation by flickering the light source **23** which is positioned on the right side of each eye of the head-mount display. Although no light source is provided on the nose-side of each frame in FIG. **3****,** a light source may be provided on every side of each frame, while also providing the line-of-sight measurement section **18.** However, care must be taken to prevent the light from any light source disposed on the left-eye (right-eye) frame from entering the opposite right eye (left eye).

Thus, by providing the line-of-sight measurement section **18** to control presentation of a visual stimulation, it is ensured that a visual stimulation is presented in the peripheral visual field of a driver. Thus, it is possible to determine whether or not attention is being paid to the peripheral visual field with a higher accuracy.

Furthermore, FIG. **22** shows a block construction diagram of a driving attention amount determination apparatus **2b** which includes the situation detection section **17** and the line-of-sight measurement section **18.** The driving attention amount determination apparatus **2b** further includes the line-of-sight measurement section **18** in addition to the construction of the driving attention amount determination apparatus **2a** (FIG. **15****)** of Embodiment 2.

When the driving attention amount determination apparatus **2b** detects the velocity of the driver's vehicle and the activation/inactivation of the head lamps of the driver's vehicle by using the situation detection section **17,** the peripheral stimulation detection section **16** changes the definitions of the central and peripheral visual fields in accordance with the velocity of the driver's vehicle and the activation/inactivation of the head lamps of the driver's vehicle as detected by the situation detection section **17.** FIG. **17** shows an exemplary central visual field **182** which is constricted based on the result of detection by the situation detection section **17.** Herein, the central visual field **182** is more constricted than the conventional viewing angle of 20°, and is defined by a range of about 16° centered around the position of the fixation point **181,** for example. Similarly to Embodiment 2, the driving attention amount determination apparatus **2b** determines an amount of attention to the peripheral visual field in accordance with the velocity of the driver's vehicle and the activation/inactivation of the head lamps of the driver's vehicle.

With respect to each of the above-described Embodiments, any process that was described by employing a flowchart can be implemented as a program to be executed by a computer. Such a computer program may be distributed on the market in the form of a product recorded on a storage medium, such as a CD-ROM, or transmitted via telecommunication lines such as the Internet.

All or some of the constituent elements composing the driving attention amount determination apparatus may be implemented as a general-purpose processor (semiconductor circuit) executing a computer program. Alternatively, they may be implemented as a special processor in which such a computer program and a processor are integrated. For example, a processor executing a computer program receives an electroencephalogram signal of a driver which is measured by the electroencephalogram measurement section **11.** Then, from the electroencephalogram signal measured based on a starting point which is the temporal point of occurrence of a visual stimulation occurring in the peripheral visual field of the driver, the processor determines an amount of attention of the driver to the peripheral visual field, and outputs a signal based on the result of determination. As a result, attention of the driver can be called.

Otherwise, as a processor executes a computer program, the processor may control the operations of the peripheral stimulation generation section **12,** the imaging section **15,** the peripheral stimulation detection section **16,** the situation detection section **17,** the line-of-sight measurement section **18,** and the like, or the processor may function as each such constituent element.

### INDUSTRIAL APPLICABILITY

The driving attention amount determination apparatus according to the present invention is useful for preventing accidents in connection with events that may occur in the peripheral visual field of the driver, e.g., a sudden intrusion of a vehicle or a rushing out of a pedestrian. In the case where it is implemented as a head-mount display type apparatus, it is also applicable to safety assistance while riding a bicycle or while walking.

### REFERENCE SIGNS LIST

- **1, 1a, 2, 2a, 2b, 3, 100**: driving attention amount determination apparatus
- **11**: electroencephalogram measurement section
- **12**: peripheral stimulation generation section
- **13**: attention amount determination section
- **14**: output section
- **15**: imaging section
- **16**: peripheral stimulation detection section
- **17**: situation detection section
- **18**: line-of-sight measurement section

## Claims

1. A driving attention amount determination apparatus comprising:
an electroencephalogram measurement section for measuring an electroencephalogram signal of a driver;
an attention amount determination section for determining an amount of attention of the driver to a peripheral visual field by utilizing an event-related potential in the electroencephalogram signal, the event-related potential being based on a starting point which is a time point of occurrence of a visual stimulation occurring in the peripheral visual field of the driver; and
an output section for calling attention of the driver by outputting a signal based on a result of the determination.

2. The driving attention amount determination apparatus of claim 1, wherein the attention amount determination section determines the amount of attention in accordance with an amplitude level of the event-related potential of the electroencephalogram signal based on a starting point which is the time point of occurrence of the visual stimulation.

3. The driving attention amount determination apparatus of claim 2, wherein the attention amount determination section determines that the amount of attention is small if an amplitude of a P300 component of the event-related potential is smaller than a predetermined threshold value, the P300 component being a positive component in a zone from 300 milliseconds to 600 milliseconds based on a starting point which is the time point of occurrence of the visual stimulation.

4. The driving attention amount determination apparatus of claim 3, wherein the output section outputs the signal to the driver when the attention amount determination section determines that the amount of attention is small.

5. The driving attention amount determination apparatus of claim 3, wherein,
the attention amount determination section determines that the amount of attention is large if the amplitude of the P300 component of the event-related potential is greater than the predetermined threshold value; and
when it is determined that the amount of attention is large, the output section does not output the signal to the driver.

6. The driving attention amount determination apparatus of claim 1, wherein the attention amount determination section determines the amount of attention in accordance with a correlation coefficient between a prestored template and the electroencephalogram signal measured based on a starting point which is the time point of occurrence of the visual stimulation.

7. The driving attention amount determination apparatus of claim 1, wherein the output section outputs at least one of: a video signal for presenting a text or a symbol on a screen for presenting information; and an audio signal to be output from a loudspeaker for outputting an audio.

8. The driving attention amount determination apparatus of claim 2, further comprising a peripheral stimulation generation section for generating the visual stimulation in the peripheral visual field of the driver.

9. The driving attention amount determination apparatus of claim 1, further comprising:
an imaging section for capturing a video of a front of a vehicle being driven by the driver; and
a peripheral stimulation detection section for, from the captured video, detecting the time point of occurrence of the visual stimulation occurring in the peripheral visual field, wherein,
from the peripheral stimulation detection section, the attention amount determination section receives information identifying the detected time point of occurrence of the visual stimulation.

10. The driving attention amount determination apparatus of claim 9, further comprising a line-of-sight measurement section for measuring a line of sight of the driver, wherein
the peripheral stimulation detection section detects whether the visual stimulation has occurred in the peripheral visual field or not in accordance with the captured video and the line of sight of the driver at the time point of occurrence of the visual stimulation measured by the line-of-sight measurement section.

11. The driving attention amount determination apparatus of claim 9, further comprising a situation detection section for detecting a velocity or head lamp activation of the vehicle, wherein,
in accordance with a result of detection by the situation detection section, the peripheral stimulation detection section detects whether the visual stimulation is in the peripheral visual field or not.

12. The driving attention amount determination apparatus of claim 9, wherein, if a difference in timing of occurrence between a visual stimulation detected in the peripheral visual field and a visual stimulation detected in the central visual field is equal to or less than a predetermined value,
the attention amount determination section excludes, from the subject of analysis, any event-related potential of the electroencephalogram signal with respect to the visual stimulation detected in the peripheral visual field.

13. The driving attention amount determination apparatus of claim 8, wherein the peripheral stimulation generation section causes the visual stimulation in the peripheral visual field of the driver to be generated with a timing of occurrence having a difference from a timing of occurrence of the visual stimulation occurring in the central visual field of the driver, the difference being equal to or greater than a predetermined value.

14. A method of determining an amount of driving attention, comprising the steps of:
measuring an electroencephalogram signal of a driver;
determining an amount of attention of the driver to a peripheral visual field by utilizing an event-related potential in the electroencephalogram signal, the event-related potential being based on a starting point which is a time point of occurrence of a visual stimulation occurring in the peripheral visual field of the driver; and
calling attention of the driver by outputting a signal based on a result of the determination.

15. A computer program, to be executed by a computer, for determining an amount of driving attention,
the computer program causing the computer to execute the steps of:
receiving an electroencephalogram signal of a driver;
determining an amount of attention of the driver to a peripheral visual field by utilizing an event-related potential in the electroencephalogram signal, the event-related potential being based on a starting point which is a time point of occurrence of a visual stimulation occurring in the peripheral visual field of the driver; and
outputting a signal based on a result of the determination,
thereby calling attention of the driver.
